# EUROPEAN PATENT APPLICATION

(11) **EP 4 349 453 A1**
(43) Date of publication of application: **10.04.2024**
(21) Application number: 22200132.3
(22) Date of filing: 06.10.2022
(51) Int. Cl.: B01D 53/22, C12M 1/00

(54) **INSTALLATION AND PROCESS FOR PRODUCING BIOMETHANE**

(71) Applicant: L'AIR LIQUIDE, SOCIETE ANONYME POUR L'ETUDE ET L'EXPLOITATION DES PROCEDES GEORGES CLAUDE, 75007 Paris (FR)
(72) Inventor: BREMOND, Ulysse, 78350 Jouy-en-Josas (FR); VALENTIN, Solène, 38360 Sassenage (FR); BARRAUD, François, 38360 Sassenage (FR); OLLIER, Jeremy, 38360 Sassenage (FR); MAENG, Min Ho, Newark, DE, 19702 (US); KULKARNI, Sudhir, Newark, DE, 19702 (US); Fang, Shu, 19702 Newark, DE (FR)
(74) Representative: Air Liquide

(57) **Abstract**

The present invention relates to an installation (1) for producing biomethane from organic waste feedstock comprising a biogas production system (2) comprising at least one production vessel (4) configured for containing digestate (6), and a biogas upgrading system (3) comprising a device for increasing the pressure of biogas (81) and a membrane separation unit (5), the membrane separation unit (5) comprising i) a first membrane separation stage (7) configured to produce a first permeate (9) and a first retentate (11), the first membrane separation stage (7) comprising a first stage gas separation membrane more permeable to carbon dioxide than to methane, a first feed inlet (13), a first permeate outlet (15), and a first retentate outlet (17), and ii) a second membrane separation stage (19) configured to produce a second permeate (21) and a second retentate (23), the second membrane stage (19) comprising a second stage gas separation membrane more permeable to carbon dioxide than to methane, a second feed inlet (25), a second permeate outlet (27), and a second retentate outlet (29), the second feed inlet (25) being in fluid communication with the the first retentate outlet (17), the installation (1) further comprising a feed conduit (8) connecting the biogas production system (2) to the biogas upgrading system (3) for directing biogas produced by the biogas production system (2) to the biogas upgrading system (3) and a recirculation conduit (31) connecting the second permeate outlet (27) to the at least one production vessel (4) of the biogas production system (2) for injecting the second permeate (21) into the digestate (6).

## Description

The invention relates to the field of green energy production and recovery techniques.

More particularly, the invention relates to an installation for producing biomethane from organic waste feedstock comprising a biogas production system comprising at least one production vessel configured for containing digestate, and a biogas upgrading system comprising a device for increasing the pressure of biogas and a membrane separation unit, the membrane separation unit comprising i) a first membrane separation stage configured to produce a first permeate and a first retentate, the first membrane separation stage comprising a first stage gas separation membrane more permeable to carbon dioxide than to methane, a first feed inlet, a first permeate outlet, and a first retentate outlet, and ii) a second membrane separation stage configured to produce a second permeate and a second retentate, the second membrane stage comprising a second stage gas separation membrane more permeable to carbon dioxide than to methane, a second feed inlet, a second permeate outlet, and a second retentate outlet, the second feed inlet being in fluid communication with the first retentate outlet, the installation further comprising a feed conduit connecting the biogas production system to the biogas upgrading system for directing biogas produced by the biogas production system to the biogas upgrading system.

Full-scale anaerobic digestion (AD) is carried out in large tanks or vessels, where the biological conversion of organic carbon into mineral carbon (biogas) occurs in the absence of oxygen. Biogas can be subject to various forms of valorization, the dominant one being its use in a Cogeneration Heat and Power (CHP) unit. However, more and more biogas is upgraded into biomethane via different technologies including adsorption on solids, chemical or physical absorption (water scrubbing or amines), membrane separation or cryogenic separation. Among these solutions, membrane separation is the most installed biogas upgrading technology in recent years in the European Union.

State of the art for membrane processes are systems with two- to four- stage membranes that allow more than 95% recovery of the methane present in the biogas. Biogas is first cooled down to remove water condensate, then filtered using systems such as activated carbon or pressure swing adsorption with thermal oxidizer systems to eliminate pollutants such as H₂S and volatile organic compounds (VOCs) and then compressed, typically to 5 - 15 bar. The compressed and cleaned biogas is finally fed to the membrane separation system. Via permeation, major gas compounds that are biomethane, carbon dioxide, nitrogen and oxygen are separated. Carbon dioxide and part of oxygen permeate through the membrane preferentially into the lower pressure permeate side (typically 1 - 2 barA). To recover most of the biomethane, the permeate from the 2nd stage membrane is sent directly back to the compressor. In the case where additional membrane stages are present, the final retentate from these stages can also be recycled and sent back to the compressor.

In recent years, several studies have focused on the injection of CO₂ inside digestate to enhance anaerobic digestion performances. Positive results were obtained and carbon dioxide bubbling into anaerobic digester has been notably reported to double AD sludge methane production rate, increase in methane yield of food waste by 13%, increase in methane yield of sewage sludge by 6 to 12%, and allow the digester to operate at higher organic loading rate while maintaining high methane production performances.

Precise biological mechanisms uptake of carbon dioxide and its conversion to methane remains unclear and several hypotheses have been put forward, based on the injection of pure or high-grade CO₂:
It enhances the hydrogenotrophic methanogenesis pathway (CO₂ + 4H₂ → CH₄ + 2H₂O).
It enhances homoacetogenesis (4H₂ + 2CO₂ → CH₃COOH + 2H₂O) and subsequently acetoclastic methanogenesis pathway (CH₃COOH → CH₄ + CO₂).
It boosts volatile fatty acids formation by acting on digestate pH and alkalinity (CO₂ dissolved in digestate could subsequently decrease pH of the anaerobic digestion and increase the alkalinity and buffering capacity of the digestate).
A combination of these different pathways.

The above described systems, however, present a number of drawbacks. For the state-of-the-art membrane-upgrading unit, no additional benefit is obtained on anaerobic digestion process as the gas recycling occurs within the upgrading system. Moreover, the injection of high-grade CO₂ inside the digester requires to bring on-site, store and inject externally produced CO₂, which has limited interest from an economic and environmental point of view. The injection of high-grade CO2 or even the recirculation of a part of the off-gas rich in CO₂ (above 90%v/v) recovered at the outlet of the membrane-upgrading unit would dilute biogas, reducing membrane efficiency and increasing operational expenditure for the upgrading unit.

It is amongst the objects of the present invention to mitigate the aforementioned disadvantages. The invention proposes a solution to increase methane recovery from organic feedstocks as well as to enhance process stability.

To this end, the installation according to the invention, which also conforms to the generic definition given in the above preamble, is essentially characterized in that the installation comprises a recirculation conduit connecting the second permeate outlet to the at least one production vessel of the biogas production system for injecting the second permeate into the digestate, the recirculation conduit having a first end in fluid communication with the second permeate outlet and a second end in fluid communication with the at least one production vessel of the biogas production system, the at least one production vessel comprising a lower volume configured for storing digestate and an upper volume configured for containing biogas, the second end of the recirculation conduit being connected to at least one injection inlet located on the at least one production vessel, said at least one injection inlet being fluidly connected to the lower volume of said at least one production vessel for injecting the second permeate into the digestate.

The disclosed installation may include one or more of the following aspects:
- a recycle conduit having a first end in fluid communication with the recirculation conduit and a second end in fluid communication with the feed conduit for recycling the second permeate back into the biogas upgrading system,
- the membrane separation unit further comprises a third membrane separation stage configured to produce a third permeate and a third retentate, the third membrane separation stage comprising a third stage gas separation membrane more permeable to carbon dioxide than to methane, a third feed inlet, a third permeate outlet, and a third retentate outlet, the third feed inlet being in fluid communication with the first permeate outlet,
- the third retentate outlet is connected to the recirculation conduit by a retentate recirculation conduit for injecting the third retentate into the digestate contained in the at least one production vessel of the biogas production system,
- the third retentate outlet is connected to the recycle conduit by a retentate recycle conduit for reinjecting the third retentate into the feed conduit,
- at least one valve, in particular at least one automated or controlled valve, for directing the third retentate towards the recirculation conduit and/or the recycle conduit,
- the membrane separation unit further comprises a fourth membrane separation stage configured to produce a fourth permeate and a fourth retentate, the fourth membrane separation stage comprising a fourth stage gas separation membrane more permeable to carbon dioxide than to methane, a fourth feed inlet, a fourth permeate outlet, and a fourth retentate outlet, the fourth feed inlet in fluid communication with the third retentate outlet,
- the fourth retentate outlet is connected to the recirculation conduit by a retentate recirculation conduit for injecting the fourth retentate into the digestate contained in the at least one production vessel of the biogas production system,
- the fourth retentate outlet is connected to the recycle conduit by a retentate recycle conduit for reinjecting the fourth retentate into the feed conduit,
- at least one valve, in particular at least one automated or controlled valve, for directing the fourth retentate towards the recirculation conduit and/or the recycle conduit,
- at least one valve, in particular at least one automated or controlled valve, for directing the second permeate towards the recirculation conduit and/or the recycle conduit,
- a compressor or a blower is located in the recirculation conduit,
- a heat exchanger is located downstream of the compressor or the blower and upstream of the biogas production system, the heat exchanger being coupled to a heat source, such as a boiler, and/or a cold source, such as a chiller.

The invention also relates to a process for producing biomethane from organic waste feedstock comprising:
∘ anaerobic digestion of organic waste feedstock in at least one production vessel; comprised in a biogas production system to produce a biogas stream and a digestate;
∘ compressing the biogas stream to provide a compressed biogas stream;
∘ separating the compressed biogas stream with a first stage gas separation membrane into a first permeate stream and a first retentate stream;
∘ separating the first retentate stream with a second stage gas separation membrane into a second permeate stream and a second retentate stream; and
∘ injecting at least a portion of the second permeate stream into the digestate contained in the at least one production vessel of the biogas production system.

The disclosed process may include one or more of the following aspects:
- at least a portion of the second permeate stream is mixed with the biogas stream,
- all of the second permeate stream is injected into the digestate contained in the at least one of the production vessel,
- the process further comprises:
   ∘ separating the first permeate stream with a third stage gas separation membrane into a third permeate stream and a third retentate stream; and
   ∘ injecting third retentate stream into the digestate contained in the at least one production vessel,
- The third retentate stream is combined with the second permeate stream before being injected into the digestate contained in the at least one production vessel,
- the combined second permeate stream and the third retentate stream is compressed before being injected into the digestate contained in the at least one production vessel,
- the process further comprises:
   ∘ separating the first permeate stream with a third stage gas separation membrane into a third permeate stream and a third retentate stream,
   ∘ separating the third retentate stream with a fourth stage gas separation membrane into a fourth permeate stream and a fourth retention stream; and
   ∘ injecting the fourth retentate stream into the digestate contained in the at least one production vessel,
- the fourth retentate stream is combined with the second permeate stream before being injected into the digestate contained in the at least one production vessel,
- the combined second permeate stream and the fourth retentate stream is compressed before being injected into the digestate contained in the at least one production vessel.

The present invention allows the permeate gas and/or retentate gas from the membrane separation system to be recirculated directly into the existing tank(s) of the biogas plant and/or via a satellite reactor where digestate is pumped in. Particularly, it takes advantage of the existing gas recirculation scheme of the membrane upgrading system. Furthermore, the injection of CO₂ in the anaerobic digestion process can be achieved without the drawbacks of high-grade CO₂ injection or CO₂-rich off-gas recirculation, thereby improving methane yield and ensuring higher stability of biological processes. In addition, bubbling generated by the CO₂ injection can provide additional mixing of digestate especially in the case where gas injection occurs in an existing tank with a mechanical mixing system.

The installation and process according to the invention can be applied to all wet or dry digester systems: Continuous stirred tank reactor, upflow anaerobic sludge blanket, induced blanket reactor, fluidized bed reactor, internal circulation reactor, anaerobic fixed bed reactor, anaerobic sequencing batch reactor, anaerobic membrane bioreactor, batch digesters (for instance, garage or covered pit), horizontal or vertical plug-flow and all other types of innovative digester systems. In addition, they can be applied to single or two-stage anaerobic digestion, regardless of the biogas plant size or capacity, provided that the biogas plant is coupled to a membrane upgrading system.

Further features and advantages of the present invention will become apparent from the following detailed description, taken in conjunction with the accompanying drawings, in which:
Figure 1 shows a schematic diagram of a first example of the structure and operation of an installation according to the invention.
Figure 2 shows a schematic diagram of a second example of a detail of the structure and operation of an installation according to the invention.
Figure 3 shows a schematic diagram of a third example of the structure and operation of an installation according to the invention.
Figure 4 shows a schematic diagram of a fourth example of the structure and operation of an installation according to the invention.
Figure 5 shows a schematic diagram of a fifth example of the structure and operation of an installation according to the invention.
Figure 6 shows a schematic diagram of a sixth example of the structure and operation of an installation according to the invention.
Figure 7 shows a schematic diagram of a seventh example of the structure and operation of an installation according to the invention.
Figure 8 shows a schematic diagram of an eighth example of the structure and operation of an installation according to the invention.
Figure 9 shows a schematic diagram of a ninth example of a detail of the structure and operation of an installation according to the invention.

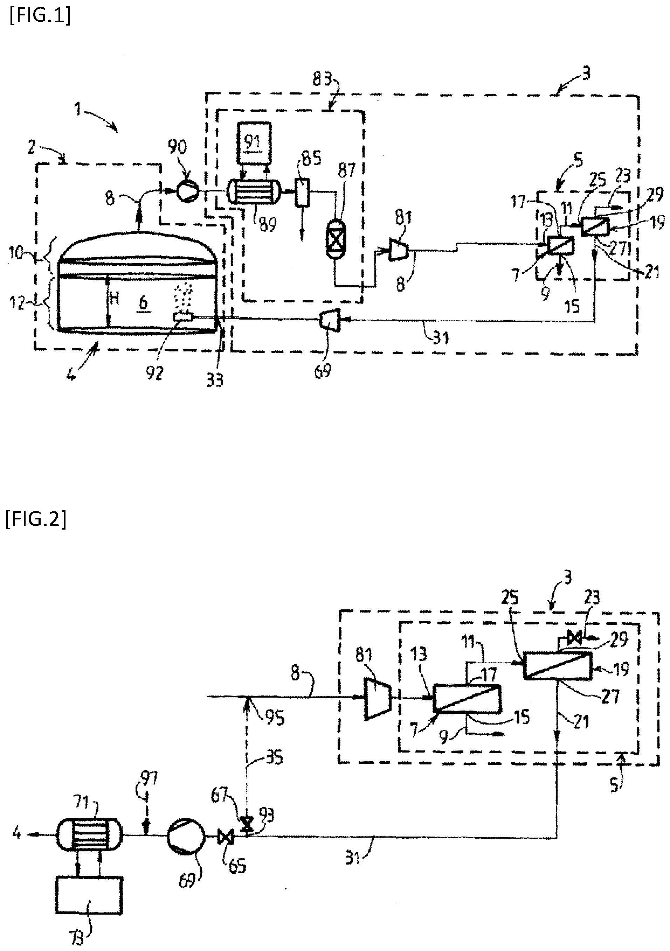

Figure 1 represents an installation 1 for producing biomethane from organic waste feedstock. Examples of organic waste feedstock include agricultural residues, industrial wastewater, animal manure, biowaste, sewage sludge, roadside grass or energy/sequential crops. It should be apparent to those skilled in the art that organic waste feedstock may be any organic matter capable of being broken down by microorganisms in the absence of oxygen.

The installation 1 comprises a biogas production system 2 for producing biogas by anaerobic digestion of organic waste feedstock and biogas upgrading system 3 for producing biomethane from the biogas produced from the biogas production system 2.

The biogas production system 2 includes a production vessel 4, in particular a digester, which is an enclosed reactor in which anaerobic digestion or methanation takes place. The production vessel 4 comprises a lower volume 12 configured for containing digestate 6 and an upper volume 10 configured for containing biogas produced during anaerobic digestion of organic waste feedstock.

As used herein, the term "digestate" refers to a mixture of organic feedstock, water, minerals and microorganisms. It is composed of liquid and solid fractions that can be obtained after phase separation. Digestate can be located in biogas plant feeding pipes, hydrolysis tank, digester tank, post-digester tanks and storage tank.

As used herein, the term "production vessel" refers to a tank, unit or reactor in which biogas is produced by anaerobic digestion of biodegradable or organic matter.

The lower volume 12 of the production vessel 4 corresponds to the lower part of the interior volume of the production vessel 4 occupied by digestate 6 up to a height H of the production vessel 4. The lower volume 12 may range from about 10 % to about 90 % of the total interior volume of the production vessel 4, for example from about 20 % to about 80 % of the total interior volume of the production vessel 4. Depending on the total interior volume and/or the height of the production vessel, the height H delimiting the lower volume 12 of the production vessel 4 may be at least about 50 % to 95 % of the height of the production vessel 4, for example from about 60 % to 90 % or from about 80 % to about 85 % or from about 85 % to 90 % of the height of the production vessel 4.

The digestate 6 occupying the lower volume may be a whole digestate containing solid and liquid fractions or a liquid digestate containing liquid fraction after solid-liquid separation of whole digestate to remove almost all of the solid fraction therefrom.

In a particular embodiment, the production vessel 4 may comprise a mixing mechanism/system or a mechanical mixing equipment such as mechanical mixers or recirculation pumps. for mixing the digestate 6, continuously or intermittently, within the production vessel 4 during anaerobic digestion of organic waste feedstock. Mixing in the production vessel 4 ensures homogeneity of the digestate, even distribution of temperature and pH in the anaerobic digestion environment, homogeneous microbial degradation and stable biogas production.

The upper volume 10 of the digester 6 corresponds to the headspace or the upper part of the interior volume of the digester 6 above digestate and contains raw biogas produced during anaerobic digestion of the organic waste feedstock.

The biogas production system 2 is connected to the biogas upgrading system 3 via a feed conduit 8. The feed conduit 8 directs the biogas produced by the biogas production system 2 towards the biogas upgrading system 3. The feed conduit 8 has a first end in fluid communication with the headspace of the production vessel 4 and a second end in fluid communication with the biogas upgrading system 3. The raw biogas produced by the biogas production system 2 is thus fed into the biogas upgrading system 3 via the feed conduit 8.

The biogas upgrading system 3 comprises a device for increasing the pressure of biogas or a pressure-increasing device 81, in particular a compressor, and a membrane separation unit 5 downstream of the pressure-increasing device 81. In a preferred embodiment, the biogas upgrading system 3 may include a pre-treatment unit 83 upstream of the pressure-increasing device 81 for filtering out moisture and contaminants such as hydrogen sulfide, Volatile Organic Compounds (VOCs), or siloxanes in the raw biogas. The pre-treatment unit 83 may comprise a water removal device 85 such as a demister or an activated carbon adsorption filter 87. In some embodiments, the pre-treatment unit 83 may further comprise a heat exchanger 89, in particular coupled to a chiller 91, to cool down the raw biogas. The biogas upgrading system 3 may further comprise a blower 90 upstream of the pre-treatment unit 83 for extracting the raw biogas from the headspace of the production vessel and feeding the same into the biogas upgrading system3, in particular the pre-treatment unit 83.

The membrane separation unit 5 comprises two membrane separation stages connected in series, each stage comprising one or more membranes.

The term "membrane separation stage" as employed herein refers to a unit comprising a feed inlet, one or more membranes connected in series and/or in parallel, and a permeate outlet and a retentate outlet. A gas stream enters through the feed inlet, separated into two streams across one or more membranes connected in series and/or in parallel, and produces a permeate and a retentate which exit through the permeate outlet and the retentate outlet respectively.

A first membrane separation stage 7 is configured to separate the biogas stream, in particular the compressed biogas stream or the pre-treated and compressed biogas stream, into a first permeate 9 and a first retentate 11. The first membrane separation stage 7 comprises a first stage gas separation membrane, in particular two or more first stage separation membranes, more permeable to carbon dioxide than to methane, a first feed inlet 13, a first permeate outlet 15, and a first retentate outlet 17. The first feed inlet 13 is connected downstream of the pressure-increasing device 81, in particular a compressor, for introducing the compressed biogas stream into the first membrane separation stage 7. The compressed biogas stream is separated into a first permeate stream 9 and a first retentate stream 11, which exit via the first permeate outlet 15 and the first retentate outlet 17 respectively.

A second membrane separation stage 19 is configured to separate the first retentate stream 11 into a second permeate stream 21 and a second retentate stream 23. The second membrane stage 19 comprises a second stage gas separation membrane, in particular two or more second stage separation membranes, more permeable to carbon dioxide than to methane, a second feed inlet 25, a second permeate outlet 27, and a second retentate outlet 29. The second feed inlet 25 is connected to the first retentate outlet 17 for introducing the first retentate stream 11 into the second membrane separation stage 19.

The installation 1 further comprises a recirculation conduit 31 which connects the second permeate outlet 27 to the production vessel 4 of the biogas production system 2 for injecting the second permeate stream 21 into digestate 6 as a recirculation gas. The recirculation conduit 31 has a first end in fluid communication with the second permeate outlet 27 and a second end in fluid communication with at least one the production vessel 4 of the biogas production system 2. In particular, the second end of the recirculation conduit 31 is connected to at least one injection inlet 33 located on the production vessel 4 and the installation is configured for injecting the second permeate 21 into digestate via this injection inlet 33.

That is to say, the at least one injection inlet 33 is fluidly connected to the lower volume 12 of said at least one production vessel 4 for injecting the second permeate 21 into the digestate 6.

The second permeate 21 can be injected into the digestate 6 directly at its exit from the injection inlet 33 but preferably via an injection device (conduit(s), nozzle(s), etc.) configured for transfering the second permeate 21 from the injection inlet 33 to a determined location(s) of the lower volume 12 of production vessel 4 containing digestate 6 (liquid/solid or liquid).

In a possible embodiment, the recirculation conduit 31 extends into the production vessel 4 via the injection inlet 33 so that the second end of the recirculation conduit 31 is located in the lower volume 12 of the production vessel 4 dedicated to the digestate 6 when the installation is in operation. This second end may comprise hole(s)/nozzle(s)/porous part(s) for injecting the second permeate 21 into digestate 6.

The second end of the recirculation conduit 31 is thus configured to allow the recirculation gas, in particular second permeate stream 21, to be injected directly into the digestate 6 contained/stored in the production vessel 4.

The term "injection inlet" as used herein refers to the location of the production vessel 4 where the recirculation conduit 31 is connected to and through which the recirculation gas 21 enters the production vessel 4.

The at least one injection inlet 33 may be located on any part of the production vessel 4, including the sidewall along the entire height of the production vessel 4, the top or roof, for example the roof of a concrete tank/production vessel, the membrane covers, or the bottom floor of the production vessel 4. The injection inlet 33 may be located on or close to the bottom surface and/or on/close to the sidewall of the production vessel 4. The injection inlet 33 may be located on the surface of the production vessel 4, the interior of which corresponds to the lower volume 12 of the production vessel 4, for example on the sidewall along the height H of the production vessel 4 occupied by digestate 6. In particular, the injection inlet 33 may be located on the sidewall of the production vessel at from about 0 % (bottom floor of the production vessel) to about 100 % of the heightH of the production vessel 4. For example, the injection inlet 33 may be located at about 10%, 20%, 30%, 40%, 50%, 60%, 70%, 80% or 90% of the height H of the production vessel 4. The injection inlet 33 may be located on the sidewall of the production vessel 4 above the height H of the production vessel 4, i.e. on the sidewall surrounding the upper volume 10.

In a particular embodiment, the injection inlet 33 is located on the surface of the production vessel 4, the interior of which corresponds to the lower volume 12 of the production vessel 4 and the second end of the recirculation conduit 31 is located at the injection inlet 33 of the production vessel 4 for injecting the second permeate 21 from the opening of the injection inlet 33.

The second end of the recirculation conduit 31 may comprise at least one injection outlet or nozzle, as a gas outlet. In particular, the at least one injection outlet or nozzle is located in the lower volume 12, i.e. inside the digestate 6, of the production vessel 4. The at least one injection outlet or nozzle is fluidly connected to the injection inlet 33 of the production vessel 4. The at least one injection outlet or nozzle may be directed towards the bottom and/or side wall (s) and/or toward the top/upper part of the production vessel 4 within the lower volume 12. Preferably, the location(s) of the at least one injection outlet or nozzle within the lower volume 12 of the production vessel 4 is(are) determined so as to ensure the maximum surface exchange between the recirculation gas 21 (the second permeate) exiting the at least one injection outlet or nozzle and the digestate 6 contained in the production vessel 4.

As illustrated in Figure 1, the recirculation conduit 31 may extend into the interior of the production vessel 4 from the injection inlet 33 and the second end of the recirculation conduit 31 is located in the lower volume 12 of the production vessel 4. The location of the second end of the recirculation conduit 31 is determined depending on the location of the recirculation gas injection inside the digestate 6. Regardless of the location of the injection inlet 33, the recirculation gas 21 is directed towards and into the digestate 6 in the production vessel 4.

The second end of the recirculation conduit 31 may comprise hole(s)/nozzle(s) and/or any type of gas injection device 92 allowing the release of gas into the digestate 6.

The recirculation conduit 31 may comprise a plurality of second ends for injecting the second permeate 21 at different locations in the production vessel 4.

Advantageously, the gas injection device 92 is designed so as to allow high surface exchange between gas (the recirculation gas 21) and liquid.

In a particular embodiment, the gas injection device 92 comprises a plurality of injection outlets or nozzles. The plurality of injection outlets is located in the lower volume 12 of the production vessel 4 and fluidly connected to the injection inlet 33 inside the production vessel 4 via one or more pipes, hoses or tubes or via at least one second end of the recirculation conduit 31. The pipes, hoses or tubes or the second end(s) of the recirculation conduit 31 extend(s) from the surface (top, bottom and/or side) of the production vessel 4 from the injection inlet 33. The plurality of injection outlet(s) or nozzle(s) may be directed towards the bottom and/or side walls and/or toward the upper part of the production vessel 4 within the lower volume 12.

The location of the (plurality of) injection outlet(s) or nozzle(s) within the lower volume 12 of the production vessel 4 may vary depending on the dimensions of the gas injection device 92 chosen for ensuring the maximum surface exchange between the recirculation gas 21 (the second permeate) exiting the (plurality of) nozzle outlet(s) and the digestate contained in the production vessel 4.

The gas injection device 92 may comprise holes, perforations or pore canals, or may be made of a porous or sintered material, in particular made of rubber, polymers, ceramic or metals. The plurality of injection outlets or nozzles may correspond to said holes, perforations or pore canals, in particular those of the porous or sintered material, in particular made of rubber, polymers, ceramic or metals.

In a particular embodiment, the gas injection device 92 may be or comprise a frame or a panel comprising or made of a plurality of pipes, in particular fluidly interconnected pipes, comprising holes, perforations or pore canals, or made of a porous or sintered material, such as rubber, polymers, ceramics or metals. The plurality of pipes, in particular fluidly interconnected pipes may be made of rubber, polymers, ceramic or metals.

The gas injection device 92, in particular the frame or panel, may extend along at least a part or the entirety of the bottom surface and/or along a part of the sidewall of the production vessel 4. The plurality of injection outlets or nozzles may be distributed along at least a part of the surface or the entire surface of the gas injection device 92. For example, the gas injection device 92 is a Poroxal^{®} injector.

Such a gas injection device allows high surface exchange between the recirculation gas 21 and digestate 6 via bubbles or micro-bubbles formation to favor gas uptake by microorganisms.

The recirculation conduit 31 may comprise at least one analyser for monitoring the quality of the recirculation gas such as gas composition and temperature. For example, the recirculation conduit 31 may comprise a CO₂ analyzer for determining the quantity of CO₂ in the recirculation gas. The recirculation conduit 31 may comprise a flow meter and/or a device for adjusting the recirculation gas flow.

In some embodiments, the installation may further comprise a compressor or a blower 69 in the recirculation conduit 31. The compressor or blower 69 is located upstream of the production vessel 4 in order to direct the second permeate stream 21 towards the production vessel 4 and inject it into the digestate 6 contained in the production vessel 4. The compressor or blower 69 allows the recirculation gas 21 to reach the adequate pressure, for example between 2 and 5 barA, and the flow required for the injection into the digestate 6.

In a variant shown in Figure 2, the installation 1 further comprises a recycle conduit 35 having a first end 93 in fluid communication with the recirculation conduit 31 and a second end 95 in fluid communication with the feed conduit 8 for recycling the second permeate 21 back into the biogas upgrading system 3. In particular, the first end 93 of the recycle conduit 35 is connected to the recirculation conduit 31 upstream of the compressor or blower 69. The second permeate stream 21 is combined with the biogas stream, in particular with the pre-treated biogas stream, in the feed conduit 8 by means of the recycle conduit 35, before being compressed in a pressure increasing device 81 prior to be introduced into the membrane separation unit 5.

In some embodiments, the installation 1 further comprises at least a set of valve(s), for example valve 65 and/or 67, in particular at least one automated or controlled valve, for directing the second permeate 21 towards the production vessel 4 via the recirculation conduit 31 and/or towards the feed conduit 8 via the recycle conduit 35. The at least a set of valve(s) may be located on the recirculation conduit 31 and/or the recycle conduit 35. In a particular embodiment, the recycle conduit 35 comprises a valve 67 that can be open for directing at least a part the second permeate stream 21 through the recycle conduit 35 towards the feed conduit 8 upstream of the pressure-increasing device 81 or closed for directing all of the second permeate stream 21 through the recirculation conduit 31 towards the digestate 6 contained in the production vessel 4. In a particular embodiment, the recirculation conduit 31 comprises a valve 65 that can be open for directing at least a part or all of the second permeate stream 21 through the recirculation conduit 31 towards the digestate 6. In a particular embodiment, the installation 1 comprises a valve 67 in the recycle conduit 35 and a valve 65 in the recirculation conduit 31. The valves 65, 67 may be open or closed simultaneously or independently for directing the second permeate stream 21 towards the production vessel 4 via recirculation conduit 31 and/or the feed conduit 8 via the recycle conduit 35. The valves may be progressive valves or flow-control valves that allow progressive opening or closing for controlling the gas flow rate.

The injection of the second permeate stream 21 into the digestate 6 may be carried out continuously or sequentially according to the optimal process conditions that would be determined for each biogas plant.

In a particular embodiment, the installation 1 may further comprise a foam mitigation system before the injection inlet 33 for mitigating foam formation during the injection of the recirculation gas (second permeate stream) into the digestate 6. The foam mitigation system may be an antifoaming agent, in particular an antifoaming liquid.

In a particular embodiment, the installation 1 may comprise a gas addition line 97 for injecting additional gas from a gas source, in particular an external gas source. The gas addition line 97 has a first end configured to be connected to an external gas source and a second end connected to the recirculation conduit 31. The external gas source may be one or more gas cylinders or gas tanks. Through the gas addition line 97, an additional gas such as oxygen, hydrogen or carbon dioxide, or any combination thereof may be introduced into the recirculation conduit 31 and combined with the recirculation gas 21, in particular the second permeate stream 21, before being injected into the digestate 6. The gas addition allows tuning of the gas composition of the recirculation gas, i.e. the second permeate stream 21, that is injected into the digestate and stimulates biogas production or desulfurization.

In some embodiments, the installation 1 may comprise a heat exchanger 71, in the recirculation conduit 31, downstream of the compressor or blower 69 and upstream of the biogas production system 2. The heat exchanger 71 may be coupled to a heat source 73, such as a boiler, and/or a cold source 73, such as a chiller. The heat exchanger 71 allows the recirculation gas, i.e. the second permeate stream 21, to reach a predetermined temperature before being injected into the digestate 6. In a particular embodiment, the heat exchanger 71 is coupled to a cold source 73 for adjusting, in particular cooling, the temperature of the recirculation gas 21. Preferably, the temperature of the recirculation gas is adjusted to about 37 °C before being injected into the digestate 6. In some embodiments, the installation 1 may comprise a heater, in particular instead of a heat exchanger 71, for adjusting, in particular heating, the temperature of the recirculation gas 21.

The installation 1 may comprise a plurality of production vessels 4. The production vessels may be a hydrolysis tank, a digester, a post-digester, storage tank and/or an injection reactor or satellite reactor. The recirculation gas can be injected directly via the recirculation conduit 31 into a single production vessel or several production vessels simultaneously or sequentially.

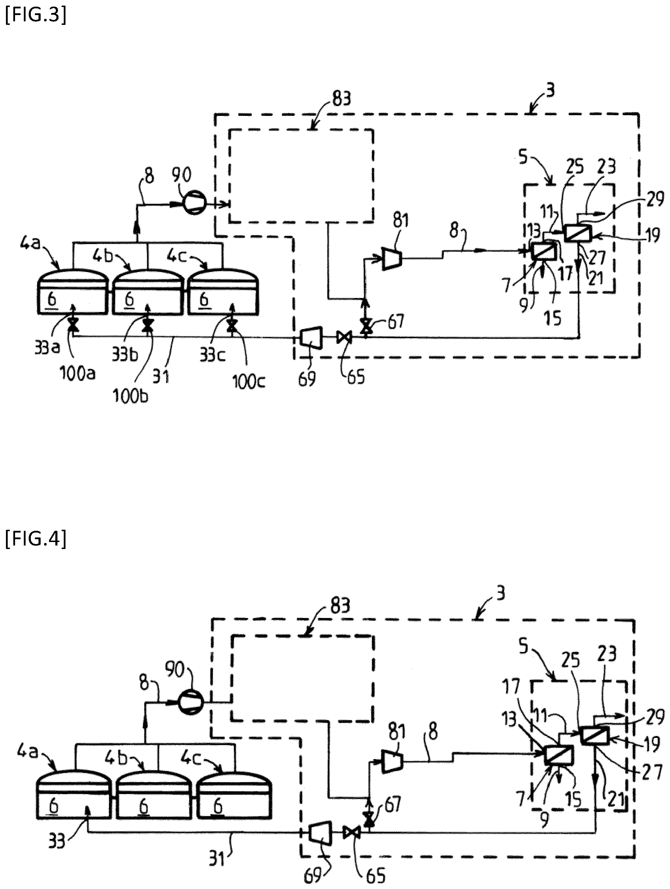

Figure 3 shows an installation 1 having three production vessels 4a, 4b, 4c, for example, a hydrolysis tank, a digester, and a storage tank. The recirculation conduit is connected to the injection inlet 33a, 33b, 33c of each of the hydrolysis tank, the digester and the storage tank located on the bottom surface. The injection inlet 33a, 33b, 33c of each of these production vessels 4a, 4b, 4c are connected in parallel to the recirculation conduit 31 via a respective valve 100a, 100b, 100c. The valves 100a, 100b, 100c pilot the second permeate stream 21 towards any one, two or all of these production vessels 4a, 4b, 4c via the recirculation conduit 31 for injecting the second permeate stream 21 into the digestate 6 contained therein, independently or simultaneously. The raw biogas produced from each of these production vessels is recovered from the headspace of each of these production vessels 4a, 4b, 4c, combined and directed towards the biogas upgrading system 3 through a feed conduit 8.

Figure 4 shows a variation of an installation 1 having three production vessels, for example, hydrolysis tank 4a, a digester 4b and a storage tank 4c. The recirculation conduit 31 is connected to the injection inlet 33 of one of the production vessels 4a, for example, the hydrolysis tank. The raw biogas produced from the hydrolysis tank, the digester, and the storage tank is recovered from the headspace of each of these production vessels 4a, 4b, 4c, combined and directed towards the biogas upgrading system 3 through a feed conduit 8.

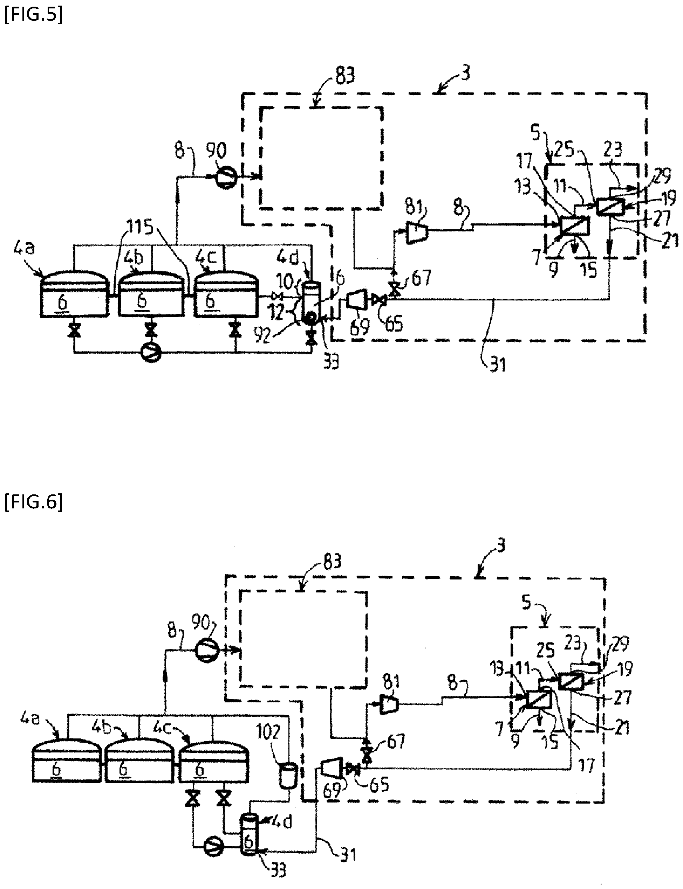

Figure 5 shows an installation 1 having four production vessels for example, a digester 4a, a post-digester 4b, a storage tank 4c and an injection reactor or satellite reactor 4d. The recirculation conduit 31 is connected to the injection reactor 4d for injecting the second permeate stream 21 into the digestate 6 contained in the injection reactor 4d. The injection reactor 4d is connected to the digester 4a, the post-digester 4b and the storage tank 4c via a pipe system and a pump system for introducing the digestate 6 from any of the production vessels 4a, 4b, 4c into the injection reactor 4d, in particular for pumping and circulating digestate 6 into and out of the injection reactor 4d. The pipe system and the pump system may comprise one or more valves.

The pipe system and the pump system may be configured so that the digestate 6 enters into the injection reactor 4d through an inlet and exits out of the injection reactor 4d through an outlet, the inlet and the outlet being located apart from each other. For example, the inlet may be located near the bottom surface of the injection reactor 4d and the outlet may be located near the top of the lower volume 12 but below the height H of the injection reactor 4d. In another example, the inlet may be located near the top of the lower volume 12 but below the height H of the injection reactor 4d and the outlet may be located near the bottom surface of the injection reactor 4d.

The injection reactor 4d, in particular the inlet and/or outlet of the injection reactor 4d for digestate 6, may be connected to any one or two or all of the production vessels 4a, 4b, 4c. For example, the inlet of the injection reactor 4d may be connected to all of the production vessels 4a, 4b, 4c and the outlet of the injection reactor 4d may be connected to all of the production vessels 4a, 4b, 4c. In an example illustrated in Figure 5, the inlet of the injection reactor 4d is connected to all of the production vessels 4a, 4b, 4c and the outlet of the injection reactor 4d is connected to the production vessels 4c. In another example, the outlet of the injection reactor 4d may be connected to all of the production vessels 4a, 4b, 4c and the inlet of the injection reactor 4d may be connected to the production vessels 4c.

The pipe system and the pump system for pumping and circulating digestate 6 into and out of the injection reactor 4d may be configured to allow the digestate 6 to circulate through one or two of all of the production vessels 4a, 4b, 4c via the bottom surface or the sidewall within the lower volume 12, in particular within the lower half of the height H, of said production vessel(s) 4a, 4b, 4c and/or via the overflow pipe(s) 115 connecting said production vessels 4a, 4b, 4c.

The digestate 6 contained in any one or two or all of the production vessels 4a, 4b, 4c may be circulated or pumped into and out of the injection reactor 4d through a pipe system and a pump system and/or an overflow system 115 between the production vessels 4a, 4b, 4c. For example, the digestate contained in the digester 4a , the post-digester 4b and the storage tank 4c is pumped and fed into the injection reactor 4d and then pumped out of the injection reactor 4d into the storage tank 4c for circulating the digestate 6 through the storage tank 4c.

The injection reactor 4d may be connected to an external source containing digestate such as lagoon or tanker truck containing digestate. The pipe system and the pump system allow the digestate 6 contained in one or more of the digester 4a , the post-digester 4b, the storage tank 4c, and /or an external source containing digestate to be pumped and circulated into and out of the injection reactor 4d. The digestate 6 may be circulated continuously or intermittently or periodically. The pumping system allows the flow of the digestate to go upward or downward inside the injection reactor 4d.

The injection reactor 4d may be in any shape. In particular, the injection reactor 4d is a column. The size of the injection reactor 4d depends on the volume of treatment in connection with the associated biogas plant. The injection reactor 4d may comprise a mechanical mixing equipment. Preferably, the injection reactor 4d is insulated and the temperature therein can be regulated. The injection reactor 4d may be operated under mesophilic, thermophilic or even higher temperature conditions, for example up to 100 °C. The injection reactor 4d may also be operated under pressure or vacuum conditions. All types of gas injection device 92 may be coupled with the injection reactor 4d, in particular a gas injection device that allows for high surface exchange between the injected gas, i.e. recirculation gas 21, and the digestate 6. The gas injection device 92 is connected to the injection inlet 33 that is connected to the recirculation conduit 31. The gas injection device 92 is located at the bottom of the injection reactor 4d. The injection reactor 4d may further comprise one or more organs for adding solid and/or liquid additives and for regulating pH of the digestate 6. The raw biogas produced from the digester 4a, the post-digester 4b, the storage tank 4c and the injection reactor 4d is recovered from the headspace of each of these production vessels, combined and directed towards the biogas upgrading system 3 through a feed conduit 8.

In an example shown in Figure 6, the injection reactor 4d is connected to one of the three production vessels, for example, a storage tank 4c, via a pipe system and a pump system for introducing the digestate 6 from the storage tank 4c into the injection reactor 4d, in particular for circulating the digestate 6 into and out of the injection reactor 4d. The installation 1 may further comprise a scrubber 102 coupled to the injection reactor 4d for recovering the nitrogen from the raw biogas produced in the injection reactor 4d. In particular, the scrubber 102 is a column. The nitrogen recovery can be optimized by washing the raw biogas produced by the injection reactor 4d with a strong acidic solution, such as sulphuric acid or nitric acid. The scrubber 102 is connected to the headspace or upper volume 10 above the digestate 6 of the injection reactor 4d for injecting the nitrogen-enriched raw biogas produced in the injection reactor 4d into the scrubber 102. In a particular example, the raw biogas produced from the injection reactor 4d is injected into the bottom of the scrubber column 102. The raw biogas produced from the digester 4a, the post-digester 4b, the storage tank 4c is combined with the raw biogas recovered from the scrubber 102 and directed towards the biogas upgrading system 3 through a feed conduit 8.

A scrubber 102 may be coupled to any one or more of the production vessels 4a, 4b, 4c and/or 4d.

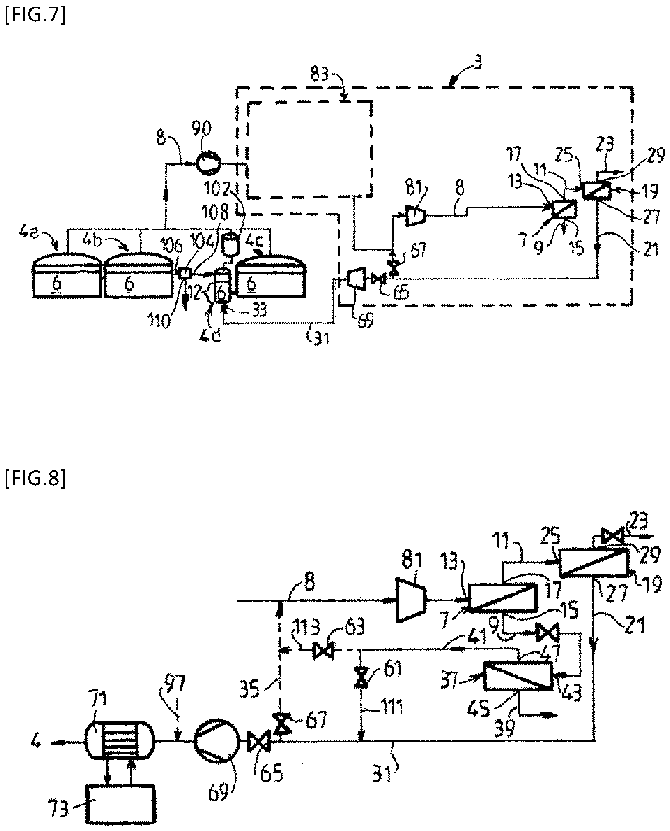

In another example shown in Figure 7, the installation 1 comprises a phase separator 104 for separating digestate 6 into liquid and solid fractions. The phase separator 104 has an inlet 106 connected to a production vessel, for example, the post-digester 4b for introducing the digestate 6, in particular whole digestate, from the post-digester 4b, one liquid outlet 108 for providing the liquid fraction of digestate or liquid digestate and one solid outlet 110 for providing the solid fraction of digestate or solid digestate. The liquid outlet 108 is connected to the injection reactor 4d for introducing the liquid digestate into the injection reactor 4d. The lower volume 12 of the injection reactor 4d may be connected to a production vessel 4c, for example a storage tank for transferring the liquid digestate 6 thereinto.

The inlet 106 of the phase separator 104 may be connected to any one or more of the production vessels 4a, 4b, 4c containing digestate 6, for introducing the digestate 6 containing liquid and solid fractions into the phase separator 104.

As illustrated in Figure 8, the membrane separation unit 5 may comprise a third membrane separation stage 37. The third membrane separation stage 37 is configured to separate the first permeate stream 9 into a third permeate 39 and a third retentate 41. The third membrane separation stage 37 comprises a third stage gas separation membrane, in particular two or more third stage gas separation membranes, more permeable to carbon dioxide than to methane, a third feed inlet 43, a third permeate outlet 45, and a third retentate outlet 47. The third feed inlet 43 is connected to the first permeate outlet 15 for introducing the first permeate stream 9 into the third membrane separation stage 37. The third retentate outlet 47 is fluidly connected to the recirculation conduit 31 via a retentate recirculation conduit 111 for injecting the third retentate stream 41 into the digestate 6 contained in the at least one production vessel 4 of the biogas production system 2. The third retentate stream 41 being directed to the recirculation conduit 31 is a recirculation gas. The retentate recirculation conduit 111 directs the third retentate stream 41 towards the recirculation conduit 31, preferably upstream of the compressor or blower 69. The retentate recirculation conduit 111 may be located downstream or upstream of the recycle conduit 35. At least a part or all of the third retentate stream 41 may be directed towards the recirculation conduit 31 while all of the second permeate stream 21 is directed towards the recycle conduit 35 upstream of the retentate recirculation conduit 111 and mixed with the raw or pre-treated biogas stream in the feed conduit 8. Preferably, the retentate recirculation conduit 111 is located upstream of the recycle conduit 35 and the third retentate stream 41 directed towards the retentate recirculation conduit 111 is combined with the second permeate stream 21 in the recirculation conduit 31 before being injected into the digestate 6 contained in at least one production vessel 4. The third retentate stream 41 and the second permeate stream 21, are combined as a recirculation gas in the recirculation conduit 31 and injected into the digestate 6 in one combined stream. The recirculation gas, the combined second permeate stream 21 and the third retentate stream 41, may be compressed or blowed before being injected into the digestate 6 contained in the at least one production vessel 4.

The third retentate outlet 47 may be fluidly connected to the recycle conduit 35 by a retentate recycle conduit 113 for directing the third retentate 41 towards the feed conduit 8. The third retentate stream 41 is reinjected into the feed conduit 8 and thus reinjected into the biogas upgrading system 3, in particular combined with the raw biogas stream or the pre-treated biogas stream. In a particular embodiment, the third retentate stream 41 is combined with the second permeate stream 21 in the recycle conduit 35 before being reinjected into the feed conduit 8 and combined with the raw biogas stream, in particular with the pre-treated biogas stream. In a particular embodiment, the third retentate stream 41 is directed towards the recycle conduit 35 and reinjected into the feed conduit 8 without being combined with the second permeate stream 21, in particular in the case where all of the second permeate stream 21 is directed towards the recirculation conduit 31 and injected into the digestate 6 contained in a production vessel 4.

The third retentate outlet 47 may be fluidly connected to both the recirculation conduit 31 via the retentate circulation conduit 111 and the recycle conduit 35 via the retentate recycle conduit 113.

The installation 1 may further comprise at least one valve 61 and/or 63, in particular at least one automated or controlled valve, located on the retentate recirculation conduit 111 and/or the retentate recycle conduit 113, for directing the third retentate 41 towards the recirculation conduit 31 and/or the recycle conduit 35. In a particular embodiment, the retentate recirculation conduit 111 comprises a valve 61 that can be open for directing at least a part of the third retentate stream 41 towards the recirculation conduit 31 or closed for directing all of the third retentate stream 41 towards the feed conduit 8. In a particular embodiment, the retentate recycle conduit 113 comprises a valve 63 that can be open for directing at least a part or all of the third retentate stream 41 towards the feed conduit 8. In a particular embodiment, the installation 1 comprises a valve 61 in the retentate recirculation conduit 111 and a valve 63 in the retentate recycle conduit 113. The valves 61, 63 may be open or closed simultaneously or independently for directing the third retentate stream 41 towards the recirculation conduit 31 and/or the feed conduit 8. The introduction of the third retentate stream 41 into the recirculation conduit 31 may be carried out continuously or intermittently according to the optimal process conditions that would be determined for each biogas plant. The third retentate stream 41 is mixed with the second permeate stream 21 in the recirculation conduit 31 before being injected into the digestate 6. The injection of the recirculation gas stream, i.e. the combined stream of the third retentate 41 and the second permeate 21, into the digestate 6 may be carried out continuously or intermittently according to the optimal process conditions that would be determined for each biogas plant.

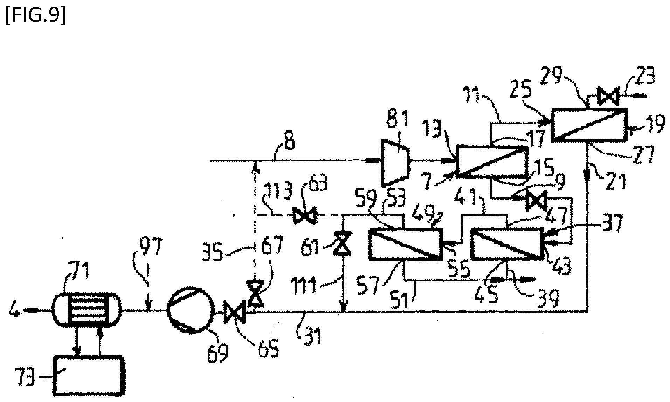

As illustrated in Figure 9, the membrane separation unit 5 may further comprise a fourth membrane separation stage 49. The fourth membrane separation stage 49 is configured to separate the third retentate stream 41 into a fourth permeate 51 and a fourth retentate 53. The fourth membrane separation stage 49 comprises a fourth stage gas separation membrane, in particular two or more fourth stage gas separation membranes, more permeable to carbon dioxide than to methane, a fourth feed inlet 55, a fourth permeate outlet 57, and a fourth retentate outlet 59. The fourth feed inlet 55 is connected to the third retentate outlet 47, thus in fluid communication with the third retentate outlet 47, for introducing the third retentate stream 41 into the fourth membrane separation stage 49. The fourth retentate outlet 59 is fluidly connected to the recirculation conduit 31 by a retentate circulation conduit 111 for injecting the fourth retentate 53 into the digestate 6 contained in the at least one production vessel 4 of the biogas production system 2. The fourth retentate stream 53 being directed to the recirculation conduit 31 is a recirculation gas. The retentate recirculation conduit 111 directs the fourth retentate stream 53 towards the recirculation conduit 31, preferably upstream of the compressor or blower 69. The retentate recirculation conduit 111 may be located downstream or upstream of the recycle conduit 35. At least a part or all of the fourth retentate stream 53 may be directed towards the recirculation conduit 31 while all of the second permeate stream 21 is directed towards the recycle conduit 35 upstream of the retentate recirculation conduit 111 and mixed with the raw or pre-treated biogas stream in the feed conduit 8. Preferably, the retentate recirculation conduit 111 is located upstream of the recycle conduit 35 and the fourth retentate stream 53 directed towards the retentate recirculation conduit 111 is combined with the second permeate stream 21 in the recirculation conduit 31 before being injected into the digestate 6 contained in at least one production vessel 4. The fourth retentate stream 53 and the second permeate stream 21, are combined as a recirculation gas in the recirculation conduit 31 and injected into the digestate 6 in one combined stream. The recirculation gas, the combined second permeate stream 21 and the fourth retentate stream 53, may be compressed or blowed before being injected into the digestate 6 contained in the at least one production vessel 4.

The fourth retentate outlet 59 may be fluidly connected to the recycle conduit 35 by a retentate recycle conduit 113 for directing the fourth retentate 53 towards the feed conduit 8. The fourth retentate stream 53 is reinjected into the feed conduit 8 and thus reinjected into the biogas upgrading system 3, in particular combined with the raw biogas stream or the pre-treated biogas stream. In a particular embodiment, the fourth retentate stream 53 is combined with the second permeate stream 21 in the recycle conduit 35 before being reinjected into the feed conduit 8 and combined with biogas stream, in particular with the pre-treated biogas stream. In a particular embodiment, the fourth retentate stream 53 is directed towards the recycle conduit 35 and reinjected into the feed conduit 8 without being combined with the second permeate stream 21, in particular in the case where all of the second permeate stream 21 is directed towards the recirculation conduit 31 and injected into the digestate contained in a production vessel 4.

The fourth retentate outlet 59 may be fluidly connected to both the recirculation conduit 31 via the retentate circulation conduit 111 and the recycle conduit 35 via the retentate recycle conduit 113.

The installation 1 may further comprise at least one valve 61 and/or 63, in particular at least one automated or controlled valve, located on the retentate recirculation conduit 111 and/or the retentate recycle conduit 113, for directing the fourth retentate 53 towards the recirculation conduit 31 and/or the recycle conduit 35. In a particular embodiment, the retentate recirculation conduit 111 comprises a valve 61 that can be open for directing at least a part of the fourth retentate stream 53 towards the recirculation conduit 31 or closed for directing all of the fourth retentate stream 53 towards the feed conduit 8. In a particular embodiment, the retentate recycle conduit 113 comprises a valve 63 that can be open for directing at least a part or all of the fourth retentate stream 53 towards the feed conduit 8. In a particular embodiment, the installation 1 comprises a valve 61 in the retentate recirculation conduit 111 and a valve 63 in the retentate recycle conduit 113. The valves 61, 63 may be open or closed simultaneously or independently for directing the fourth retentate stream 53 towards the recirculation conduit 31 and/or the feed conduit 8. The introduction of the fourth retentate stream 53 into the recirculation conduit 31 may be carried out continuously or intermittently according to the optimal process conditions that would be determined for each biogas plant. The injection of the recirculation gas stream, i.e. the combined stream of the fourth retentate 53 and the second permeate 21, into the digestate 6 may be carried out continuously or intermittently according to the optimal process conditions that would be determined for each biogas plant.

The present invention is also directed to a process for producing biomethane that can be implemented by the above-described installation.

## Claims

1. Installation (1) for producing biomethane from organic waste feedstock comprising:
a) a biogas production system (2) comprising at least one production vessel (4) configured for containing digestate (6);
b) a biogas upgrading system (3) comprising a device for increasing the pressure of biogas (81) and a membrane separation unit (5), the membrane separation unit (5) comprising:
i) a first membrane separation stage (7) configured to produce a first permeate (9) and a first retentate (11), the first membrane separation stage (7) comprising a first stage gas separation membrane more permeable to carbon dioxide than to methane, a first feed inlet (13), a first permeate outlet (15), and a first retentate outlet (17); and
ii) a second membrane separation stage (19) configured to produce a second permeate (21) and a second retentate (23), the second membrane stage (19) comprising a second stage gas separation membrane more permeable to carbon dioxide than to methane, a second feed inlet (25), a second permeate outlet (27), and a second retentate outlet (29), the first retentate outlet (17) being in fluid communication with the second feed inlet (25); and
c) a feed conduit (8) connecting the biogas production system (2) to the biogas upgrading system (3) for directing biogas produced by the biogas production system (2) to the biogas upgrading system (3); and
d) a recirculation conduit (31) connecting the second permeate outlet (27) to the at least one production vessel (4) of the biogas production system (2) for injecting the second permeate (21) into the digestate (6),
wherein the recirculation conduit (31) has a first end in fluid communication with the second permeate outlet (27) and a second end in fluid communication with the at least one production vessel (4) of the biogas production system (2),
wherein the at least one production vessel (4) comprises a lower volume (12) configured for storing digestate and an upper volume (10) configured for containing biogas, the second end of the recirculation conduit (31) being connected to at least one injection inlet (33) located on the at least one production vessel (4), said at least one injection inlet (33) being fluidly connected to the lower volume (12) of said at least one production vessel (4) for injecting the second permeate (21) into the digestate (6).

2. Installation according to claim 1, further comprising a recycle conduit (35) having a first end in fluid communication with the recirculation conduit (31) and a second end in fluid communication with the feed conduit (8) for recycling the second permeate (21) back into the biogas upgrading system (3).

3. Installation according to claim 1 or 2, wherein the membrane separation unit (5) further comprises a third membrane separation stage (37) configured to produce a third permeate (39) and a third retentate (41), the third membrane separation stage comprising a third stage gas separation membrane more permeable to carbon dioxide than to methane, a third feed inlet (43), a third permeate outlet (45), and a third retentate outlet (47), the third feed inlet (43) being in fluid communication with the first permeate outlet (15).

4. Installation according to claim 3, wherein the third retentate outlet (47) is connected to the recirculation conduit (31) by a retentate recirculation conduit (111) for injecting the third retentate (41) into the digestate (6) contained in the at least one production vessel (4) of the biogas production system (2).

5. Installation according to claim 3 or 4, wherein the third retentate outlet (47) is connected to the recycle conduit (35) by a retentate recycle conduit (113) for reinjecting the third retentate (41) into the feed conduit (8).

6. Installation according to claim 5, further comprising at least one valve (61, 63), in particular at least one automated or controlled valve, for directing the third retentate (41) towards the recirculation conduit (31) and/or the recycle conduit (35).

7. Installation according to claim 3, wherein the membrane separation unit (5) further comprises a fourth membrane separation stage (49) configured to produce a fourth permeate (51) and a fourth retentate (53), the fourth membrane separation stage (49) comprising a fourth stage gas separation membrane more permeable to carbon dioxide than to methane, a fourth feed inlet (55), a fourth permeate outlet (57), and a fourth retentate outlet (59), the fourth feed inlet (55) being in fluid communication with the third retentate outlet (47).

8. Installation according to claim 7, wherein the fourth retentate outlet (59) is connected to the recirculation conduit (31) by a retentate recirculation conduit (111) for injecting the fourth retentate (53) into the digestate (6) contained in the at least one production vessel (4) of the biogas production system (2).

9. Installation according to claim 7 or 8, wherein the fourth retentate outlet (59) is connected to the recycle conduit (35) by a retentate recycle conduit (113) for reinjecting the fourth retentate (53) into the feed conduit (8).

10. Installation according to claim 9, further comprising at least one valve (61, 63), in particular at least one automated or controlled valve, for directing the fourth retentate (53) towards the recirculation conduit (31) and/or the recycle conduit (35).

11. Installation according to any one of claims 1 to 10, further comprising at least one valve (65, 67), in particular at least one automated or controlled valve, for directing the second permeate (21) towards the recirculation conduit (31) and/or the recycle conduit (35).

12. Installation according to any one of claims 1 to 11, further comprising a gas addition line (97) having a first end and a second end, the first end being configured to be connected to a gas source and the second end connected to the recirculation conduit (31).

13. Process for producing biomethane from organic waste feedstock comprising:
- anaerobic digestion of organic waste feedstock in at least one production vessel (4) comprised in a biogas production system (2) to produce a biogas stream (8) and a digestate (6);
- compressing the biogas stream (8) to provide a compressed biogas stream;
- separating the compressed biogas stream (8) with a first stage gas separation membrane (7) into a first permeate stream (9) and a first retentate stream (11);
- separating the first retentate stream (11) with a second stage gas separation membrane (19) into a second permeate stream (21) and a second retentate stream (23); and
- injecting at least a portion of the second permeate stream (21) into the digestate (6) contained in the at least one production vessel (4) of the biogas production system (2).

14. Process according to claim 13, further comprising:
- separating the first permeate stream (9) with a third stage gas separation membrane (37) into a third permeate stream (39) and a third retentate stream (41); and
- injecting the third retentate stream (41) into the digestate (6) contained in the at least one production vessel (4).

15. Process according to claim 14, wherein the third retentate stream (41) is combined with the second permeate stream (21) before being injected into the digestate (6) contained in the at least one production vessel (4).

16. Process according to claim 13, further comprising:
- separating the first permeate stream (9) with a third stage gas separation membrane (37) into a third permeate stream (39) and a third retentate stream (41);
- separating the third retentate stream (41) with a fourth stage gas separation membrane (49) into a fourth permeate stream (51) and a fourth retention stream (53); and
- injecting the fourth retentate stream (53) into the digestate (6) contained in the at least one production vessel (4).

17. Process according to claim 16, wherein the fourth retentate stream (53) is combined with the second permeate stream (21) before being injected into the digestate (6) contained in the at least one production vessel (4).
